# EUROPÄISCHE PATENTANMELDUNG

(11) **EP 3 366 224 A1**
(43) Veröffentlichungstag der Anmeldung: **29.08.2018**
(21) Anmeldenummer: 17157732.3
(22) Anmeldetag: 23.02.2017
(51) Int. Cl.: A61B 17/00, A61B 90/00

(54) **VERSCHLUSSEINRICHTUNG FÜR EINE ÖFFNUNG IM HERZEN EINES PATIENTEN**

(71) Anmelder: Berlin Heart GmbH, 12247 Berlin (DE)
(72) Erfinder: Menon, Ares K., 12209 Berlin (DE); Matthes, Michael, 15345 Altlandsberg (DE)
(74) Vertreter: Pfenning, Meinig & Partner mbB

(57) **Zusammenfassung**

Die Erfindung bezieht sich auf eine Verschlusseinrichtung (1) für eine Öffnung (5) in einem Herz eines Patienten mit einem am Körpergewebe des Herzens im Bereich der Öffnung (5) befestigbaren Ring (3, 3a, 3b) mit einer Ringöffnung und mit einem Verschlusselement (2), das zum Verschließen der Ringöffnung in diese einbringbar ist. Es ist zudem vorgesehen, dass das Verschlusselement (2) wenigstens einen integrierten Sensor (6, 10, 11, 16, 17, 18) und/oder wenigstens einen integrierten Aktor (17, 18) aufweist. Die in oder an dem Verschlusselement (2) vorgesehenen Elemente können in verschiedenartiger Weise mit Überwachungs- und Steuergeräten verbunden sein und zusammenwirken.

## Beschreibung

Die Erfindung liegt auf dem Gebiet der Feinwerktechnik, der Elektronik und Signalverarbeitung sowie auf dem Gebiet der Medizintechnik und lässt sich mit besonderem Vorteil bei einer implantierten Verschlusseinrichtung für eine Öffnung im Herzen eines Patienten verwenden.

Oft wird bei Patienten mit bestimmten Herzdefekten eine Pumpe zur Unterstützung der Herzfunktion eingesetzt. Eine derartige Pumpe wird häufig mittels einer künstlich eingebrachten Öffnung mit einem Herzventrikel, beispielsweise dem linken Herzventrikel, verbunden, so dass eine Entlastung des Herzens durch die implantierte oder außerhalb des Körpers betriebene Pumpe erfolgen kann.

Bei manchen Patienten erholt sich unter dem Einfluss der Herzunterstützung das Herz, so dass bei Erfüllung bestimmter Entwöhnungskriterien die Pumpe wieder entfernt werden kann. Dazu wird die Pumpe oder der Pumpenanschluss aus der Öffnung der Herzkammer bzw. einem dort befestigten Nahtring herausgezogen, und die somit frei werdende Ringöffnung im Nahtring muss verschlossen werden. Zum Verschließen eines solchen Nahtrings sind verschiedene Verschlusselemente bereits bekannt, die möglichst dichtend in den Nahtring eingeschoben werden.

Der vorliegenden Erfindung liegt die Aufgabe zugrunde, eine Verschlusseinrichtung für eine Öffnung in einem Herzen eines Patienten mit zusätzlichen Funktionen auszustatten.

Die Aufgabe wird gemäß der Erfindung durch eine Verschlusseinrichtung mit den Merkmalen des Patentanspruchs 1 gelöst. Die Patentansprüche 2 bis 11 beziehen sich auf Ausführungsformen der Erfindung. Die Patentansprüche 12 und 13 beziehen sich auf eine implantierbare Einrichtung mit einer Verschlusseinrichtung nach einem der Ansprüche 1 bis 11, und der Patentanspruch 14 gibt ein Verfahren zum Betrieb einer implantierbaren Vorrichtung mit einem Verschlusselement an.

Die Erfindung bezieht sich demnach auf eine Verschlusseinrichtung für eine Öffnung in einem Herz eines Patienten mit einem am Körpergewebe des Herzens im Bereich der Öffnung befestigbaren Ring mit einer Ringöffnung und mit einem Verschlusselement, das zum Verschließen der Ringöffnung in diese einbringbar ist, dadurch gekennzeichnet, dass das Verschlusselement wenigstens einen integrierten Sensor und/oder wenigstens einen integrierten Aktor aufweist.

Ein derartiges Verschlusselement kann beispielsweise als zylindrischer oder leicht konischer Stopfen ausgebildet sein, der in eine kreisförmige Öffnung eines Rings/Nahtrings einführbar oder dichtend einpressbar ist. Grundsätzlich kann das Verschlusselement eine Einrichtung zur radialen Aufweitung in der Ringöffnung aufweisen, so dass das Verschlusselement dichtend in den Ring eingefügt werden kann.

Das Verschlusselement kann beispielsweise als Silikonstopfen ausgeführt sein oder aus einem anderen Elastomer bestehen. Im Volumen des Verschlusselements ist Raum für wenigstens einen Sensor und/oder einen Aktor. Die Sensoren oder Aktoren können aus dem Verschlusselement hinausragen, an der Außenseite des Verschlusselements angeordnet sein oder zumindest an die Außenseite des Verschlusselements angrenzen, so dass physikalische Messgrößen außerhalb des Verschlusselements durch die Sensoren erfasst werden können oder dass durch einen in das Verschlusselement integrierten Aktor auf Elemente außerhalb des Verschlusselements eingewirkt werden kann.

Der im Bereich der Öffnung des Herzens befestigbare Ring kann als Nahtring ausgebildet sein, der beispielsweise einen metallischen Teilring sowie einen vernähbaren, insbesondere aus einem textilen Gewebe bestehenden Teilring aufweist. Der vernähbare Teilring ist üblicherweise flanschartig ausgebildet, so dass er auf der Außenseite am Rand der Öffnung im Herzen anliegen kann und dort mittels eines Fadens mit dem Herzgewebe vernäht werden kann. Der metallische Teilring kann beispielsweise als Rohrstutzen ausgebildet und mit dem vernähbaren Teilring verschweißt oder verklebt sein.

Das Verschlusselement kann beispielsweise zwei axial gegeneinander komprimierbare Scheiben oder ringförmige Elemente enthalten, zwischen denen ein ringförmiges Dichtelement axial komprimierbar und damit radial aufweitbar angeordnet ist.

Das ringförmige Dichtelement kann beispielsweise aus einem Elastomer bestehen und massiv oder als Elastomerschaum ausgebildet sein, oder es kann einen Hohlraum aufweisen, der gasgefüllt oder fluidgefüllt sein kann.

Der metallische Teilring des Nahtrings ist üblicherweise komplementär zu dem Verschlusselement derart ausgebildet, dass eine fluiddichte und dauerhafte Verbindung zwischen dem Verschlusselement und dem Nahtring hergestellt werden kann.

In einer Ausführungsform der Erfindung kann vorgesehen sein, dass wenigstens ein integrierter Sensor zur Erfassung eines zeitabhängigen Drucks eingerichtet ist. Ein derartiger Sensor kann beispielsweise in einem Hohlraum des Verschlusselements angeordnet sein, wobei der Hohlraum durch eine dünne und bewegliche Membran abgeschlossen ist, die auf Druckänderungen reagiert, so dass der außerhalb des Verschlusselements wirkende Druck sich auf den Druck im Hohlraum des Verschlusselements überträgt. Der Drucksensor kann jedoch auch zum Äußeren des Verschlusselements hin unabgedeckt sein und den Druck in der Umgebung des Verschlusselements unmittelbar erfassen.

Es kann zudem in einer Ausführungsform vorgesehen sein, dass das Verschlusselement wenigstens einen Drucksensor aufweist, der einen Druck auf einer ersten Außenseite des Verschlusselementes erfasst, sowie insbesondere einen zweiten Drucksensor, der auf einer zweiten Außenseite einen Druck erfasst, wobei das Verschlusselement derart eingerichtet ist, dass in der Verschlussposition des Verschlusselementes die erste Außenseite dem Herzinneren und die zweite Außenseite dem Herzäußeren zugewandt ist.

Das Verschlusselement hat üblicherweise eine Sollposition zur Dichtung in der Ringöffnung der Verschlusseinrichtung, so dass eine Seite des Verschlusselements dazu vorgesehen ist, durch die Ringöffnung zum Herzinneren hin eingeschoben zu werden, während die gegenüberliegende Seite des Verschlusselements zur herzäußeren Seite des Rings weist. Sind zwei Drucksensoren vorgesehen, so können diese den Druck im Herzinneren einerseits und außerhalb des Herzens andererseits erfassen. Da der absolute Druck im Herzinneren jeweils nur durch Differenzbildung zum Druck in der Umgebung des Herzens ermittelt werden kann, ist eine derartige zweifache Druckmessung zur Bestimmung des absoluten Drucks im Herzen sinnvoll.

Es kann in einer weiteren Ausführungsform vorgesehen sein, dass das Verschlusselement einen Sensor oder wenigstens zwei Sensoren zur Erfassung eines Spannungssignals aufweist. Derartige Sensoren sind üblicherweise metallisch ausgebildet und wirken entweder als Antennen oder als Elektroden. Sind sie als Antennen ausgebildet, so bilden sie mit anderen elektrisch leitenden Elementen außerhalb der Verschlusseinrichtung einen Kondensator, über den eine Spannung eingekoppelt werden kann. Sind die Sensoren als Elektroden ausgebildet, so sind sie beispielsweise an der Außenseite des Verschlusselements zugänglich und können mit einem Körperfluid oder einem Körpergewebe derart in Kontakt gebracht werden, dass sie elektrische Signale erfassen können.

Beispielsweise kann vorgesehen sein, dass das Verschlusselement wenigstens eine, insbesondere wenigstens zwei Elektroden aufweist, die in wenigstens einem oder wenigstens zwei Teilbereichen einen Oberflächenbereich des Verschlusselementes, insbesondere einen zylindrischen oder teilzylindrischen Oberflächenbereich des Verschlusselementes, bilden. Jede Elektrode kann sich dabei als Zylinderabschnitt über einen Umfangsabschnitt des Verschlusselements erstrecken.

Die zylindrischen oder teilzylindrischen Elektroden können in die Oberfläche des Verschlusselements eingelegt sein und beispielsweise in dem Bereich des Verschlusselements, der durch den Ring hindurch zum Herzinneren hineinragt, Spannungspotentiale im Gewebe des Herzens erfassen. Derartige Elektroden können jedoch auch dazu dienen, Spannungsimpulse beispielsweise von einem Herzschrittmacher oder einem Defibrillator zum Herzgewebe hin zu übertragen.

Es kann in einer anderen Ausführungsform der Erfindung auch vorgesehen sein, dass das Verschlusselement wenigstens eine, insbesondere wenigstens zwei Elektroden aufweist, die jeweils einen ersten, innerhalb des Verschlusselementes angeordneten Teil und einen zweiten, aus dem Verschlusselement herausragenden Teil aufweisen, wobei der zweite Teil jeweils eine Spitze aufweist. In diesem Fall können die Spitzen der Elektroden in das Herzgewebe hineinragen und somit eine leitende Verbindung zum Herzgewebe herstellen.

Somit kann beispielsweise vorgesehen sein, dass ein in das Verschlusselement integrierter Aktor eine Elektrode aufweist, die mit einem zeitabhängigen Spannungssignal beaufschlagbar und insbesondere mit einem Steuergerät verbindbar ist.

Zudem kann bei einer Verschlusseinrichtung vorgesehen sein, dass wenigstens ein Sensor und/oder mindestens ein Aktor mittels eines Kabels oder einer nicht leitungsgebundenen Übertragungseinrichtung direkt oder mittels einer in die Verschlusseinrichtung, insbesondere in das Verschlusselement, integrierten Vorverarbeitungseinrichtung mit einem Steuer- oder Überwachungsgerät verbindbar ist.

Einer oder mehrere der Sensoren und/oder der Aktoren, die beispielsweise in das Verschlusselement integriert sind, können somit mittels eines Kabels mit einem Steuer- oder Überwachungsgerät verbindbar sein. Das Steuer- oder Überwachungsgerät kann dabei innerhalb oder außerhalb des Verschlusselements oder der Verschlusseinrichtung liegen, beispielsweise auch Teil der Verschlusseinrichtung sein oder auch außerhalb der Verschlusseinrichtung vorgesehen sein. Die Verbindung kann entweder mittels eines Kabels oder einer nicht leitungsgebundenen Übertragungseinrichtung realisiert sein, wobei in diesem Fall der Sensor und/oder der Aktor oder auch mehrere Sensoren und/oder Aktoren jeweils mit einer Sendeeinrichtung und/oder einer Empfangseinrichtung verbunden sind, die mit jeweils einer komplementären Sende- und/oder Empfangseinrichtung, beispielsweise über ein Funksignal oder eine andere nicht leitungsgebundene Kommunikationsverbindung, kommunizieren. Mögliche Verbindungsarten sind dabei eine Funkverbindung mittels Bluetooth oder eines ähnlichen Standards oder eine klassische Funkverbindung mit Frequenz- oder Amplitudenmodulation oder eine der bekannten digitalen Radioschnittstellen. Es ist aber auch eine Kommunikation über Lichtwellenleiter, Ultraschall, Infrarot oder andere Übertragungswege denkbar.

Zum Zweck der Signalübertragung kann in der Verschlusseinrichtung, insbesondere im Verschlusselement selbst, eine Vorverarbeitungseinrichtung vorgesehen sein, die unmittelbar mit einem Sensor oder Aktor verbunden ist, beispielsweise mittels eines Kabels, und die ihrerseits mit einem Sender oder Empfänger verbunden ist, der mit einem Steuer- oder Überwachungsgerät innerhalb oder außerhalb der Verschlusseinrichtung über eine nicht leitungsgebundene Übertragungsstrecke oder auch leitungsgebunden kommuniziert.

Eine Vorverarbeitungseinrichtung kann Signale eines Sensors unmittelbar weiterleiten oder verarbeiten, beispielsweise indem Parameter eines Zeitverlaufs der erfassten Größe ermittelt und weitergegeben werden. Die Vorverarbeitungseinrichtung kann jedoch auch jede andere denkbare Art der Datenverarbeitung oder Signalaufbereitung vornehmen, wie beispielsweise eine Verstärkung oder Abschwächung der Signale oder eine Filterung.

Eine weitere Ausgestaltung kann vorsehen, dass wenigstens ein Sensor und/oder mindestens ein Aktor mit einem Energiespeicher verbindbar oder verbunden ist, der entweder im Patientenkörper implantiert oder außerhalb des Patientenkörpers vorgesehen ist. Der Energiespeicher kann in Form einer Batterie, insbesondere einer wiederaufladbaren Batterie, innerhalb des Verschlusselements oder außerhalb des Verschlusselements, als Teil der Verschlusseinrichtung oder auch außerhalb der Verschlusseinrichtung vorliegen. Der Energiespeicher kann einen oder mehrere Sensoren oder Aktoren für ihre Funktion mit Energie versorgen. Der Energiespeicher kann zudem eine Vorverarbeitungseinrichtung mit Energie versorgen. Wenn ein Aktor als Teil eines Defibrillators ausgebildet ist, kann der Energiespeicher den Aktor mit der notwendigen Energie für einen Schockvorgang am Herzmuskel versorgen.

Es kann zudem vorgesehen sein, dass der Energiespeicher in das Verschlusselement oder in ein implantierbares/implantiertes Steuer- oder Überwachungsgerät integriert ist oder als Modul in den Patientenkörper implantiert ist. Der Energiespeicher kann von außerhalb des Patientenkörpers mittels einer Kabelverbindung oder induktiv über magnetische Wirkung durch die Haut des Patientenkörpers hindurch aufladbar sein. Der Energiespeicher kann beispielsweise auch als Teil eines Herzschrittmachers oder eines Defibrillators oder eines anderen implantierbaren Gerätes gebildet sein.

Eine weitere Ausgestaltung kann beispielsweise vorsehen, dass der Energiespeicher derart eingerichtet und angeordnet ist, dass er mittels einer außerhalb des Patientenkörpers angeordneten Induktionseinrichtung aufladbar ist.

Die Erfindung bezieht sich außer auf eine Verschlusseinrichtung der oben genannten Art auf eine implantierbare Einrichtung mit einer derartigen Verschlusseinrichtung, wobei die implantierbare Einrichtung zudem insbesondere einen Energiespeicher und/oder ein Überwachungsgerät und/oder ein Steuergerät aufweisen kann. Das Überwachungsgerät kann beispielsweise ein implantierbares Gerät zur Erfassung von Elektrokardiogrammen sein, und das Steuergerät kann als Herzschrittmacher oder als Defibrillator ausgebildet sein. Die genannten Geräte können jedoch auch unmittelbar Teil der Verschlusseinrichtung sein und beispielsweise im Verschlusselement oder am Verschlusselement angeordnet, insbesondere in das Verschlusselement integriert sein.

Besonders in diesem Fall ist die Verbindung mit Sensoren und/oder Aktoren im Verschlusselement besonders wenig aufwendig.

Ein Herzschrittmacher und/oder Defibrillator ist mit einer oder mehreren in das Verschlusselement integrierten Elektroden verbunden und entweder in das Verschlusselement integriert oder außerhalb des Verschlusselements angeordnet und mittels Kabeln mit den entsprechenden Elektroden verbunden.

Die Erfindung bezieht sich außer auf eine Verschlusseinrichtung und eine implantierbare Einrichtung auch auf ein Verfahren zum Betrieb einer implantierbaren Vorrichtung der oben erläuterten Art, wobei zudem vorgesehen ist, dass im implantierten Zustand mittels wenigstens eines in das Verschlusselement integrierten Drucksensors ein Druckverlauf in einem, insbesondere dem linken Herzventrikel gemessen und hieraus laufend ein enddiastolischer Fülldruck bestimmt wird, aber alternativ oder zusätzlich auch am linken oder rechten Vorhof oder am rechten Ventrikel, und/oder dass im implantierten Zustand mittels wenigstens einer in das Verschlusselement integrierten Elektrode ein elektrisches Signal oder ein Energieimpuls an den Herzmuskel abgegeben wird.

In einem Fall dient ein in das Verschlusselement integrierter Drucksensor zur Ermittlung des zeitabhängigen Drucks in einer Herzkammer, wobei aus dem Druckverlauf der enddiastolische Fülldruck bestimmt werden kann. Die Bestimmung des enddiastolischen Fülldrucks findet durch Verarbeitung der Daten des Druckverlaufs entweder in der Vorverarbeitungseinrichtung oder in einem Überwachungsgerät statt.

Es kann auch vorgesehen sein, dass laufend elektrische Impulse mittels einer oder mehrerer in das Verschlusselement integrierter Elektroden erfasst und verarbeitet werden und dass aus den Impulsen ermittelt wird, ob mittels eines Defibrillators ein Energieimpuls an den Herzmuskel abgegeben werden soll. Ist dies der Fall, so wird der entsprechende Energieimpuls in Form eines Spannungsstoßes mittels einer in das Verschlusselement integrierten Elektrode an den Herzmuskel abgegeben.

Zusammenfassend ist festzustellen, dass das vorliegende Konzept das Vorsehen eines oder mehrerer Sensoren und/oder eines oder mehrerer Aktoren in oder an einem Verschlusselement einer Verschlusseinrichtung ermöglicht, wobei der oder die Sensoren und/oder Aktoren mit Überwachungs- oder Steuereinrichtungen verbunden sein können, die innerhalb oder außerhalb des Verschlusselements sowie innerhalb oder außerhalb der Verschlusseinrichtung und sogar innerhalb oder außerhalb des Patientenkörpers vorgesehen sein können. Entsprechende Übertragungswege von Signalen können durch Kabel oder durch nicht leitungsgebundene Signale realisiert sein. Die Energieversorgung von Sensoren oder Aktoren kann auf verschiedenen Wegen über einen Energiespeicher, der entweder in ein Verschlusselement oder die Verschlusseinrichtung integriert sein oder außerhalb der Verschlusseinrichtung vorliegen kann, realisiert sein. Der Energiespeicher kann zudem auch zur Versorgung einer Überwachungs- und/oder Steuereinrichtung dienen und optional aufladbar, insbesondere induktiv aufladbar sein.

Die genannten Merkmale lassen sich in verschiedenen Konfigurationen unterschiedlich miteinander kombinieren, wie anhand der im Folgenden aufgelisteten und nachfolgend erläuterten Figuren dargestellt wird.

Dabei zeigen
- Fig. 1: eine Verschlusseinrichtung in einer ersten Ausführungsform im Längsschnitt,
- Fig. 2: eine Verschlusseinrichtung in einer zweiten Ausführungsform im Längsschnitt,
- Fig. 3: eine Verschlusseinrichtung in einer dreidimensionalen Ansicht,
- Fig. 4: eine weitere Ausführungsform einer Verschlusseinrichtung,
- Fign. 5-10: verschiedene Implantationsszenarien von Verschlusseinrichtungen,
- Fig. 11: eine erste Gruppe von Anschlussschemata sowie
- Fig. 12: eine zweite Gruppe von Anschlussschemata.

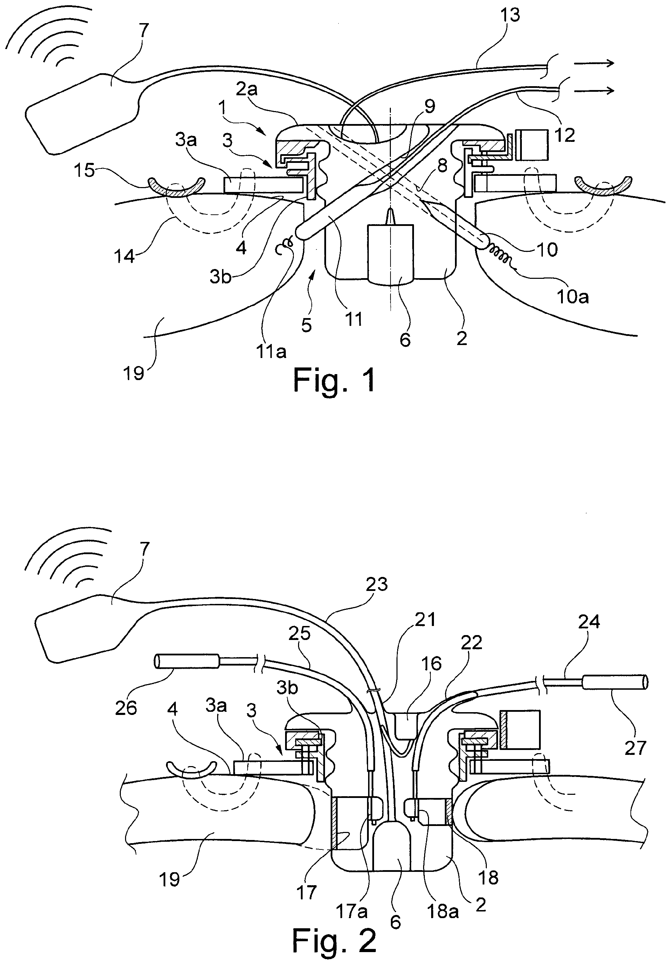

Figur 1 zeigt in einem Längsschnitt eine Verschlusseinrichtung 1 mit einem Verschlusselement 2 in Form eines Silikonstopfens sowie einem Ring 3, der am Rand 4 einer Öffnung 5 im Herzen eines Patienten befestigbar ist. In den Silikonstopfen 2 / das Verschlusselement ist ein Drucksensor 6 in dem Bereich integriert, der im implantierten Zustand der Herzkammer / dem Herzinneren, in Figur 1 im unteren Teil dargestellt, zugewandt ist. Der Drucksensor 6 erfasst zeitabhängig den Druckverlauf des Blutes im Herzen und leitet diese Signale zu einem Sender 7, der beispielsweise als Bluetooth-Sender ausgebildet sein kann und der die Signale mittels Funkwellen zu einem Bluetooth-Empfänger sendet, der innerhalb des Patientenkörpers oder außerhalb des Patientenkörpers angeordnet sein kann und der seinerseits mit einer Überwachungseinrichtung verbunden sein kann. Der Drucksensor 6 kann unmittelbar aus dem Silikonstopfen 2 herausragen oder auch in dessen Innerem vorgesehen sein, sofern die den Drucksensor bedeckende Schicht des Silikonstopfens 2 so dünn ist, dass sich Druckänderungen unmittelbar auf den Drucksensor übertragen.

Der Silikonstopfen 2 ist fluiddicht in den Ring 3 eingepresst, wobei der Ring 3 einen Nahtring 3a und einen metallischen Teilring 3b in Form eines Rohrstutzens aufweist. Der metallische Teilring 3b ist mit dem Nahtring 3a fest und insbesondere auch fluiddicht verbindbar. Der zylindrische oder konische Silikonstopfen 2 dichtet mittels einer oder mehrerer am Umfang umlaufender Dichtlippen an der Innenseite des Teilrings 3b. Der Silikonstopfen kann einen deckelartigen Vorsprung 2a aufweisen, der im eingesteckten Zustand den Teilring 3b überdeckt.

In Figur 1 ist innerhalb zweier diagonal im Inneren des Verschlusselements 2 verlaufender Kanäle 8, 9 jeweils eine Elektrode 10, 11 zu erkennen, wobei jede der Elektroden 10, 11 eine Spitze 10a, 11a zur Verankerung im Gewebe der Herzwand aufweist. Diese Elektroden 10, 11 können zur Aufnahme elektrischer Impulse aus dem Herzgewebe oder umgekehrt zur Leitung von Spannungssignalen an den Herzmuskel dienen. Die Elektroden 10, 11 sind mittels elektrischer Leitungen 12, 13 beispielsweise mit einem Überwachungsgerät verbunden. Bei dem Gerät kann es sich um einen implantierbaren Kardioverter/Defibrillator und/oder um ein Gerät für eine kardiale Resynchronisationstherapie (CRT) handeln. Die durch die Elektroden 10, 11 erfassten Signale werden am linken Ventrikel abgegriffen. Die Elektroden 10, 11 können unmittelbar durch handelsübliche Elektroden gebildet sein, die für bestimmte Herzschrittmacher von ihren Herstellern qualifiziert sind.

Der Drucksensor 6 kann so ausgebildet sein, dass er eine dünne Metallmembran aufweist, die zum Herzinneren aus dem Silikonstopfen 2 herausragt oder zumindest freiliegt und eine Druckmessung im linken Ventrikel ermöglicht. Der Drucksensor 6 kann mittels des Senders 7 die Signale bezüglich der erfassten Messgrößen an eine Überwachungs- oder Steuereinheit senden, die selbst eine Stromquelle sowie eine Sende-/ Empfangseinheit aufweist und die die Funktion hat, Sensordaten dauerhaft aufzuzeichnen, zu speichern und gegebenenfalls an ein Empfangsgerät, wie beispielsweise ein Mobiltelefon oder einen Computer oder eine körperexterne Steuereinrichtung, zu senden. Die Steuereinheit kann subkutan unter der Bauchdecke implantiert sein, so dass die Stromquelle / der Energiespeicher der Steuereinheit durch Induktion aufgeladen oder die Stromquelle ausgetauscht werden kann. Alle oder ein Teil der Kabelanbindungen bei diesem Konzept können steckbar ausgeführt sein, um die Austauschbarkeit zu erleichtern, oder als feste Verbindung, die grundsätzlich weniger störanfällig ist.

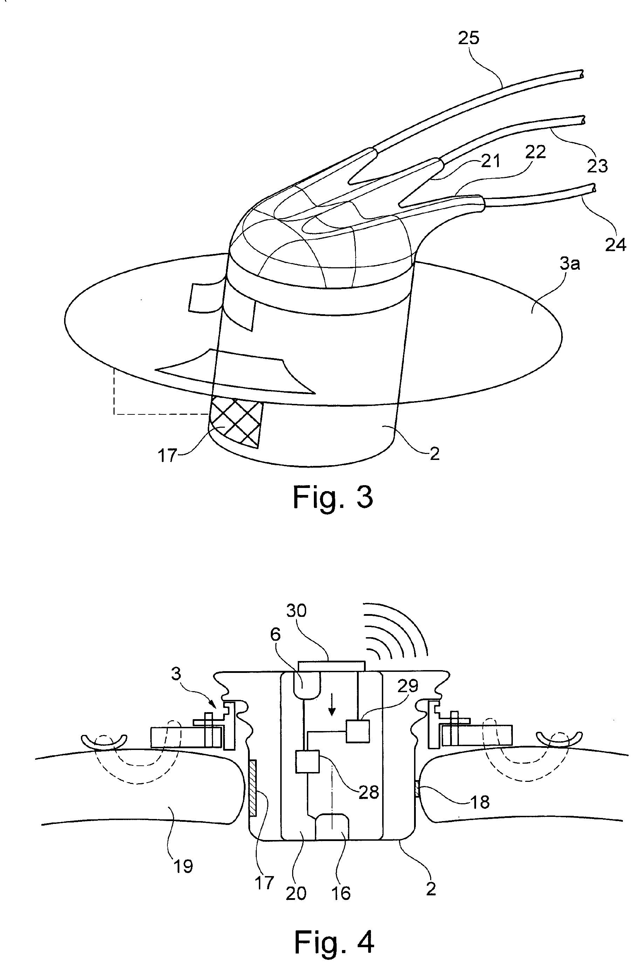

Die mit den Elektroden 10, 11 verbundenen Kabel 12, 13 können frei und ohne eine Verbindung mit dem Silikonstopfen 2 aus den im Silikonstopfen vorgesehenen Öffnungen herausragen, so dass die Elektroden 10, 11 leicht ein- und ausführbar sind. Dabei können im Silikonkörper des Stopfens 2 Stutzen vorgesehen sein, die die Kabel 12, 13 am Austritt aus dem Stopfen stützen, wobei diese Stutzen auch konisch ausgebildet und verrundet sein können. Dies ist gesondert in Figur 3 nochmals dargestellt.

Die Dichtigkeit des Verschlusselements/Silikonstopfens 2 innerhalb des Rings 3 ist dadurch gewährleistet, dass die Dichtlippen am äußeren Umfang des Stopfens an dem metallischen Ringteil 3b elastisch dichten und dass die Elektroden 10, 11 unter leichter Vorspannung in die Kanäle 8, 9 im Silikonstopfen 2 eindrückbar sind, so dass auch die Kanäle 8, 9 gegenüber dem Herzinneren abgedichtet sind. Der Drucksensor 6 kann dicht mit dem Silikonstopfen 2 verklebt oder vergossen sein.

Der Nahtring 3a ist mittels eines Fadens 14 mit dem Gewebe des Herzmuskels vernäht, wobei der Faden 14 mittels eines oder mehrerer Anker 15 an der Außenseite im Randbereich des Nahtrings fixiert ist.

Figur 2 zeigt eine Verschlusseinrichtung mit einem Silikonstopfen 2, der ähnlich wie bei der in Figur 1 dargestellten Ausführungsform in einem Ring 3 dichtend angeordnet ist. Ein Drucksensor 6 ist ähnlich wie bereits in Figur 1 dargestellt in den Silikonstopfen / das Verschlusselement 2 eingebettet.

Im Unterschied zu dem in Figur 1 dargestellten Konzept ist ein zweiter Drucksensor 16 vorgesehen, der auf der dem ersten Drucksensor 6 gegenüberliegenden Seite des Verschlusselements 2 angeordnet ist und der dazu eingerichtet ist, einen zeitabhängigen Druck außerhalb des Herzens im Patientenkörper zu erfassen, so dass Druckunterschiede und Änderungen, die außerhalb des Herzens im Thorax oder in der Atmosphäre auftreten, durch Differenzbildung der Signale der Drucksensoren 6, 16 eliminiert werden können. Der Drucksensor 16 ist ebenfalls mittels eines Kabels mit dem Sender 7 verbunden. Der Drucksensor 16 ist ebenso wie der Drucksensor 6 in den Silikonwerkstoff des Stopfens 2 eingebettet und/oder mit dem Silikonstopfen verklebt.

Es sind in dem in Figur 2 dargestellten Verschlusselement zwei Elektroden 17, 18 vorgesehen, die teilzylindrisch hülsenförmig ausgebildet sind und an der zylindrischen Oberfläche des Stopfens 2 anliegen. Jede Elektrode erstreckt sich dabei über einen Teilabschnitt des Umfangs des Stopfens. Die Elektroden 17, 18 können auch in den Stopfen 2 eingebettet sein, so dass die Oberfläche der Elektroden 17, 18 mit der Kontur des Stopfens 2 abschließt. Die Hülsenformen können auch konisch ausgebildet sein, beispielsweise für den Fall, dass der Stopfen 2 konisch zum Herzinneren hin spitz zuläuft. Im Allgemeinen wird der Stopfen 2 jedoch eine im Querschnitt runde, im Wesentlichen zylindrische und lediglich für die Passung leicht konische Form aufweisen.

Mittels der Elektroden 17, 18 wird das Gewebe der Herzwand 19 an zwei verschiedenen Stellen elektrisch kontaktiert, so dass die Elektroden 17, 18 elektrische Impulse vom Herzgewebe aufnehmen können. Die Elektroden 17, 18 sind jeweils mit Crimpverbindungen 17a, 18a mit Kabeln 19, 20 verbunden, die dichtend aus dem Verschlusselement/Silikonstopfen 2 herausgeführt sind. Zur mechanischen Stabilisierung und zur Vermeidung von Kabelbruch sind dazu an den Verschlusskörper 2 jeweils konische Kabelstützelemente 21, 22 angegossen.

Die Elektroden 17, 18 können zur Erfassung von Signalen aus dem Herzmuskel verwendet werden, um einem Herzschrittmacher oder Kardiographen zugeführt zu werden. Im vorliegenden Beispiel kann jedoch konkret auch vorgesehen sein, dass die Elektrode 17 mit einem implantierbaren Kardioverter/ Defibrillator verbunden ist. Die große Fläche der Elektrode sorgt für eine effiziente Energieverteilung im Herzen und an der Herzspitze. Die etwas kleinere Elektrode 18 kann mit einem Gerät für eine kardiale Resynchronisationstherapie (CRT) verbunden sein (linker Ventrikel). Diese Elektrode kann durch eine mögliche Drehausrichtung des Verschlusselements innerhalb des Rings 3 so positioniert werden, dass die große Elektrode möglichst auf der dem Septum zugewandten Seite positioniert ist. Dies ermöglicht eine optimale Funktion der Defibrillation.

Das hier dargestellte Konzept ist besonders für den Fall geeignet, dass ein implantierbarer Kardioverter bereits vor Einfügen des Verschlusselements implantiert wurde und in einfacher Weise mit gut platzierbaren Elektroden am Verschlusselement verbunden werden kann.

Figur 3 zeigt in einer dreidimensionalen Ansicht einen Silikonstopfen 2, wie er in Figur 2 näher beschrieben ist, mit einem Nahtring 3a, einer in die zylindrische Außenkontur des Verschlusselements 2 integrierten Elektrode 17 sowie mit drei aus dem Stopfen 2 herausgeführten Kabeln 23 (Anschluss zum Drucksensor 6), 24 (Anschluss zu einem Steuergerät für eine kardiale Resynchronisationstherapie / linker Ventrikel) und 25 (Anschluss für einen implantierbaren Kardioverter). Der Kardioverter ist in Figur 2 symbolisch durch einen Stecker 26 dargestellt, während das Steuergerät für die kardiale Resynchronisationstherapie durch einen zum Stecker 26 korrespondierenden

Stecker 27 repräsentiert ist und ebenfalls symbolisch dargestellt ist. Die Steuergeräte 26, 27 können ebenfalls implantiert sein, jedoch ist auch eine Integration in das Verschlusselement oder ein Betrieb dieser Steuergeräte außerhalb des Körpers denkbar, wobei dann die Leitungen 24, 25 entweder aus dem Patientenkörper herausgeleitet werden müssen oder an einem implantierbaren Sender enden, der die Signale mittels einer nicht leitungsgebundenen Verbindung an außerhalb des Körpers angeordnete Steuereinheiten übermittelt.

In Figur 3 sind gut die als Knickschutz wirkenden Kabelstützelemente 21, 22 in Form von konischen Ansätzen an dem Stopfen 2 zu erkennen. In einer Ausführungsform, wie sie in Figur 3 dargestellt ist, kann auch lediglich eine einzelne großfläche Elektrode 17 verwendet werden, die mit einem Herzschrittmacher verbunden wird, der sowohl die Funktion eines implantierten Kardioverters als auch eines Steuergeräts für eine kardiale Resynchronisationstherapie übernehmen kann. Die Elektrode kann dann sowohl die Sensorfunktion beim Aufnehmen von Impulsen aus dem Herzgewebe als auch die Funktion der Übertragung eines Energieimpulses auf das Herzgewebe (Pacing-Funktion) übernehmen und letztendlich auch die Defibrillationsfunktion durch Übertragung eines relativ großen Energiebetrags mittels eines elektrischen Impulses auf das Herzgewebe (Defibrillationisfunktion). Die Verbindung von Kabeln mit den jeweiligen Elektroden 17, 18 mittels Crimphülsen kann besonders günstig dann gestaltet werden, wenn die Crimphülsen selbst Teil der Elektroden / des Elektrodenblechs sind, um die Teileanzahl gering zu halten. Die Ummantelung der Kabel sollte aus einem Silikon hergestellt werden, um die Verträglichkeit der verwendeten Materialien und die Verbindung mit dem Silikonkörper des Verschlusselements zu optimieren. Crimphülsen, Kabelwerkstoff und Elektrodenmaterial sind aufgrund der elektrochemischen Spannungsreihen aufeinander abgestimmt und bestehen beispielsweise aus dem gleichen Material.

In Figur 4 ist eine Variante eines Verschlusselements 2 dargestellt, in der in den Silikonstopfen 2 eine integrierte Druckerfassungseinheit 20 eingesetzt ist, die einen oder mehrere Drucksensoren 6, 16, einen Energiespeicher in Form einer Batterie oder eines Akkus 28, eine Vorverarbeitungseinrichtung 29, beispielsweise in Form in Mikrocontrollers, und eine Sende-/Empfangseinrichtung 30 aufweist. Der Sender kann beispielsweise den Bluetooth-Standard unterstützen. Die Druckerfassungseinrichtung 20 kann in den Silikonstopfen 2 eingegossen oder eingeklebt sein.

Die Stromquelle / der Akku 28 kann aus der Einheit 20 ausgelagert und beispielsweise unter der Haut des Patienten implantiert und mit der Einheit 20 mittels eines Kabels verbunden sein, um eine induktive Aufladung zu ermöglichen. Dieses Konzept der Druckerfassungseinheit kann mit der Anordnung der Elektroden gemäß den Figuren 1, 2 oder 3 kombiniert werden. Die Anschlüsse der Elektroden sind der Einfachheit und Übersichtlichkeit halber in Figur 4 weggelassen, und es sind beispielhaft zwei teilzylindrisch hülsenförmige Elektroden 17, 18 dargestellt.

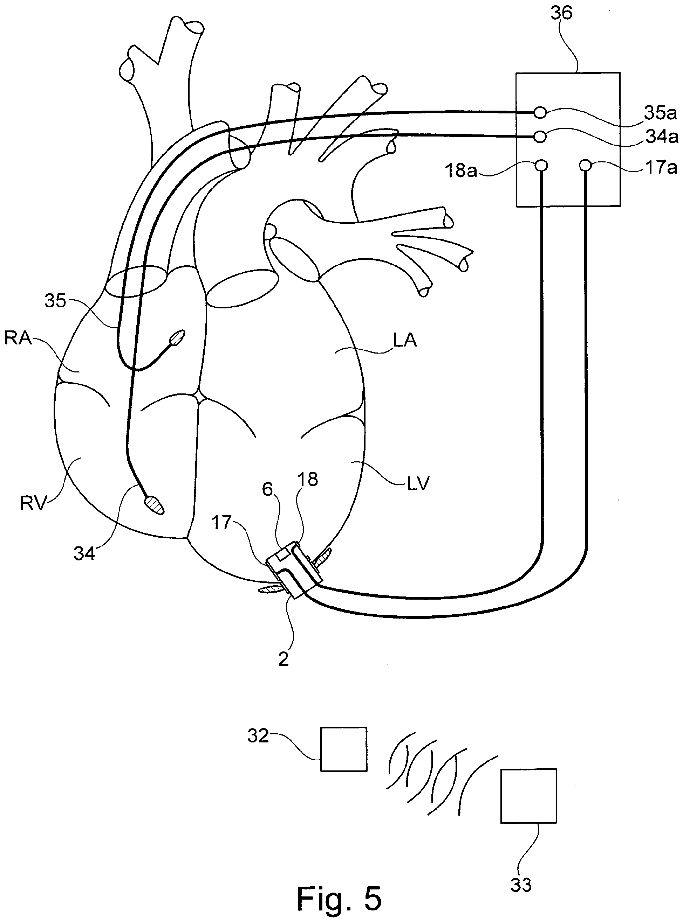

Figur 5 zeigt im Überblick ein Anschlussschema mit einer Verschlusseinrichtung, wobei die Verschlusseinrichtung ähnlich der, aber nicht notwendigerweise identisch mit der in Figur 2 dargestellten Verschlusseinrichtung aufgebaut sein kann. Es sind bei dem Verschlusselement schematisch lediglich der Silikonstopfen 2, die Elektrode 17, die Elektrode 18 und der Drucksensor 6 eingezeichnet. Bei diesem Beispiel kann der Drucksensor 6 mit einer Steuereinrichtung 32 mittels einer nicht dargestellten Leitung in Form eines Kabels oder auch über eine nicht leitungsgebundene Übertragung in Verbindung stehen. Die Steuereinrichtung 32 weist ihrerseits einen Sender und einen Empfänger auf, um eine Kommunikation mit externen Geräten 33, beispielsweise in Form eines Mobilfunkgeräts oder eines Computers, zu ermöglichen.

Die verschiedenen Teile des Herzens sind mit RA (rechtes Atrium), RV (rechter Ventrikel), LA (linkes Atrium), LV (linker Ventrikel) gekennzeichnet. Über Blutgefäße sind Elektrodenzuleitungen mit Elektroden 34 für den rechten Ventrikel, 35 für das rechte Atrium geführt, die in den genannten Herzkammern enden. Die Elektrode 34 ist mit einem Anschluss 34a eines transvenösen Herzschrittmachers 36 verbunden. Die Elektrode 35 ist mit einem Anschluss 35a des Herzschrittmachers verbunden, während die Elektrode 17 des Verschlusselements 2 mit einem Anschluss 17a des Herzschrittmachers und die Elektrode 18 des Verschlusselements 2 mit einem Anschluss 18a des Herzschrittmachers verbunden ist. Der transvenöse Herzschrittmacher 36 erhält somit die notwendigen Messdaten teilweise aus Elektroden der Verschlusseinrichtung und teilweise aus Sonden, die unabhängig davon in das Herz eingebracht sind. Eine der Elektroden 17, 18 kann zudem als Pacerelektrode dienen.

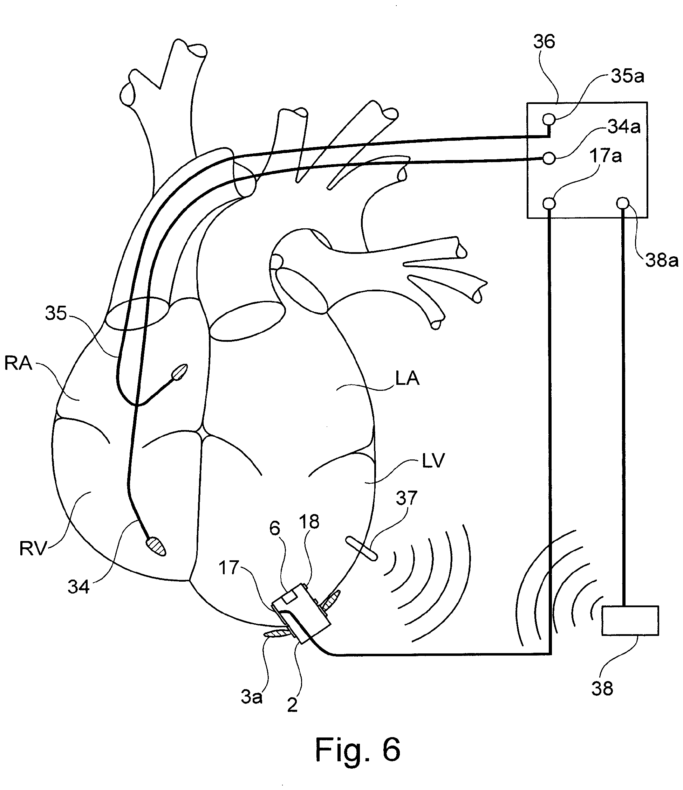

In Figur 6 ist ein Anschlusskonzept dargestellt, das ebenfalls einen transvenösen Herzschrittmacher 36 aufweist, wobei die Elektroden/Sonden 34, 35 der kardialen Resynchronisationstherapie dienen. Zudem ist eine Sonde 37 unmittelbar in den linken Ventrikel eingesetzt, die über Funk Signale an ein Modul 38 aussendet. Das Modul 38 leitet die Signale weiter an einen Anschluss 38a, der ebenfalls der kardialen Resynchronisationstherapie dient. In dem Modul 38 kann eine Vorverarbeitung der Daten stattfinden. Das Modul 38 kann auch als Empfangsmodul in den Herzschrittmacher 36 integriert sein. Zudem ist in den Herzschrittmacher ein Anschluss 17a integriert, über den Signale für eine Kardioverterfunktion von dem Herzschrittmacher zur Elektrode 17, beispielsweise auch für eine Defibrillationsfunktion, geleitet werden können.

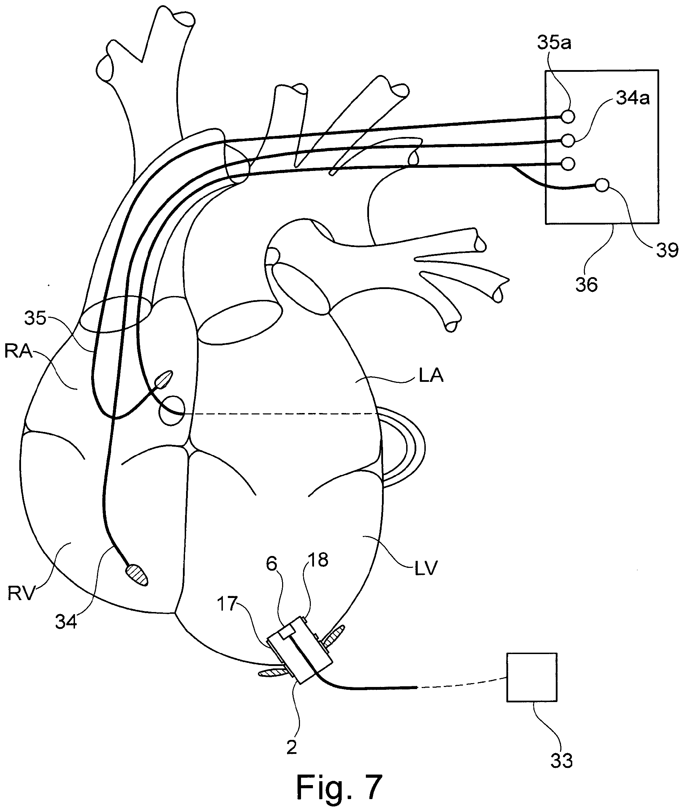

Figur 7 zeigt ein Konzept, bei dem wie in den vorangehenden Beispielen eine Sonde aus dem rechten Atrium mit dem Anschluss 35a eines Herzschrittmachers sowie eine Sonde aus dem rechten Ventrikel mit dem Anschluss 34a eines Herzschrittmachers verbunden ist. Ein Anschluss 39 des Herzschrittmachers ist mit einer Sonde im linken Ventrikel über eine Leitung verbunden, welche über den Sinus coronarius durch das rechte Atrium führt. Der Anschluss 39 kann einerseits für Signale der kardialen Resynchronisationstherapie dienen und andererseits, beispielsweise mittels eines Umschalters, für Signale eines implantierten Kardioverters für den linken Ventrikel.

Der Drucksensor 6 ist mit einem externen Gerät 33 entweder direkt über ein Kabel oder mittelbar über einen mit einem Kabel angebundenen Sender und eine Übertragungsstrecke verbunden, wie bereits in Figur 5 gezeigt.

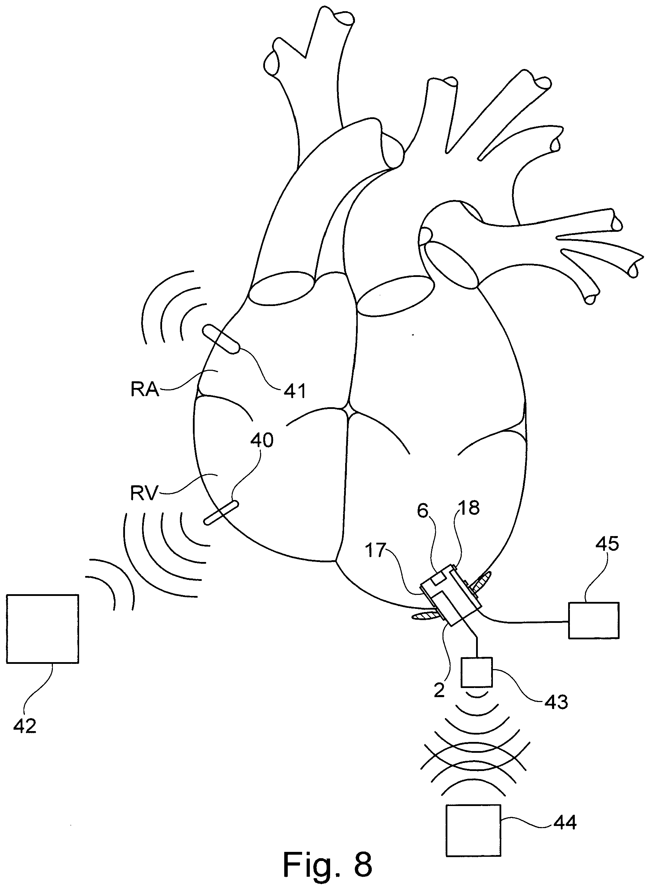

Figur 8 zeigt ein Konzept, bei dem am rechten Ventrikel RV und am rechten Atrium RA jeweils eine Sonde 40, 41 eingesetzt ist, wobei die Sonden 40, 41 mit einem Herzschrittmacher 42 per Funk kommunizieren. Der Drucksensor 6 ist mit einem Sender 43 verbunden, der über eine nicht leitungsgebundene Übertragungsstrecke mit einem externen Gerät 44 verbunden ist. Der Sender 43 kann implantierbar sein und gesondert von dem Verschlusselement 2 im Körper angeordnet werden, er kann jedoch auch in das Verschlusselement 2 integriert sein.

Die Elektrode 18 in dem Verschlusselement 2 ist mittels eines Kabels gesondert mit einem Gerät 45 verbunden, das ebenfalls implantiert sein kann und die Funktion eines implantierten Kardioverters und/oder Defibrillators übernehmen kann. Das Gerät 45 kann mit dem Herzschrittmacher 42 verbunden sein, beispielsweise leitungsgebunden oder mittels einer nicht leitungsgebundenen Verbindung. Bei dieser Variante ist keine Leitungsverlegung durch Blutgefäße des Herzens hindurch notwendig.

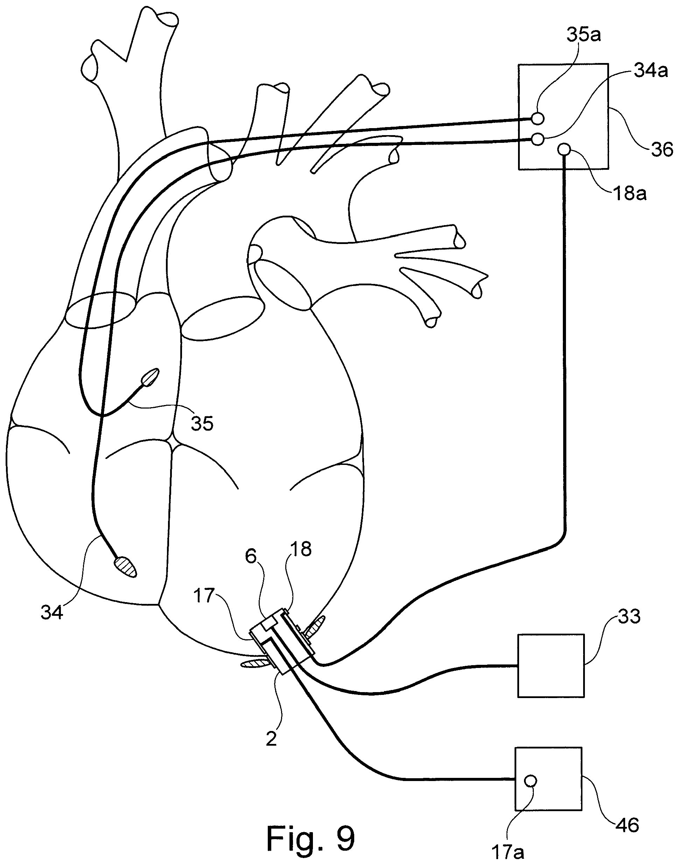

In Figur 9 ist ein Anschlusskonzept dargestellt, bei dem zwei interne Sonden 34, 35 im rechten Ventrikel und rechten Atrium mit einem Herzschrittmacher 36 mittels durch Blutgefäße verlaufender Leitungen verbunden sind, die zu den Anschlüssen 34a, 35a führen. Ein Anschluss 18a ist mit der Elektrode 18 an dem Verschlusselement 2 mittels einer Leitung verbunden. Der Herzschrittmacher 36 kann dabei außer den normalen Pacingfunktionen die Funktionen der kardialen Resynchronisationstherapie erfüllen.

Zusätzlich ist die Elektrode 17 mittels einer Leitung mit dem Anschluss 17a verbunden, der innerhalb eines implantierbaren Kardioverters/Defibrillators 46 angeordnet ist. Die Defibrillationsfunktion wird somit durch ein von dem Herzschrittmacher 36 getrenntes Gerät 46 erfüllt. Der Drucksensor 6 ist leitungsgebunden mit der Steuereinrichtung 33 verbunden.

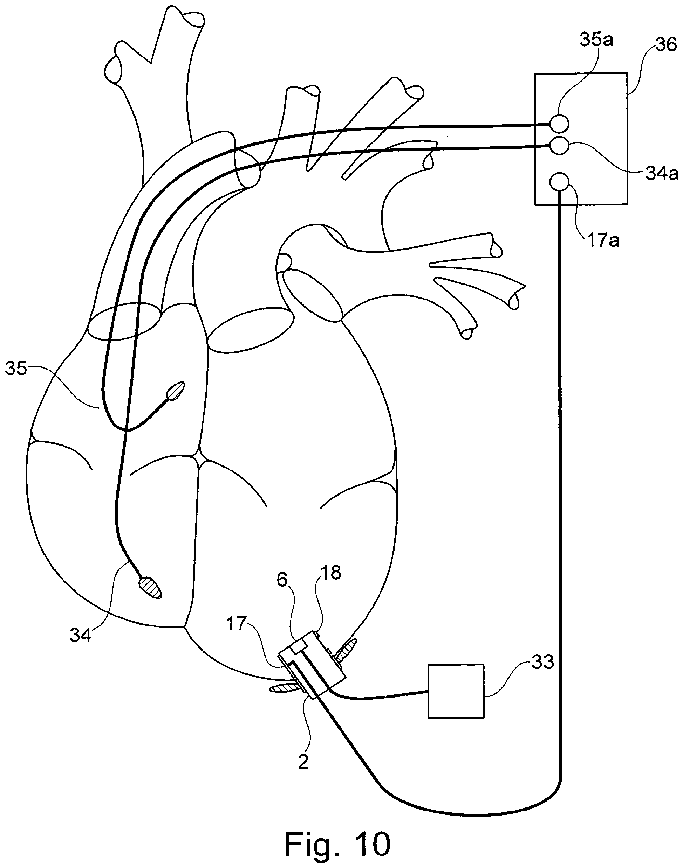

Figur 10 zeigt ein Anschlusskonzept, bei dem Sonden/Elektroden 34, 35 im rechten Ventrikel und rechten Atrium mit Anschlüssen 34a, 35a eines Geräts 36 verbunden sind und bei dem zudem eine Elektrode 17 des Verschlusselements 2 mit einem Anschluss 17a in dem Gerät 36 verbunden ist. Die Verbindungen sind leitungsgebunden hergestellt, wobei die Leitungen der Sonden 34, 35 durch Blutgefäße des Herzens hindurch verlaufen. Der Drucksensor 6 ist mit einer Steuereinrichtung 33 verbunden.

Das Gerät 36 erfüllt gleichzeitig die Funktionen eines Herzschrittmachers für eine kardiale Resynchronisationstherapie oder für eine übliche Stimulation des Herzens und andererseits die Funktion eines implantierten Kardioverters, der auch eine Defibrillationsfunktion über die Elektrode 17 ausüben kann. Der Anschluss 17a an dem Gerät 36 erfüllt somit potenziell beide Funktionen: einerseits die Zuleitung eines elektrischen Signals von der Elektrode 17 zum Gerät und andererseits die Aussendung eines elektrischen Impulses über die Zuleitung zu der Elektrode 17. Zu diesem Zweck kann innerhalb des Geräts 36 eine Umschalteinrichtung vorgesehen sein, die die Zuleitung der Elektrode 17 mit verschiedenen Eingängen oder Schaltkreisen innerhalb des Geräts 36 verbindet.

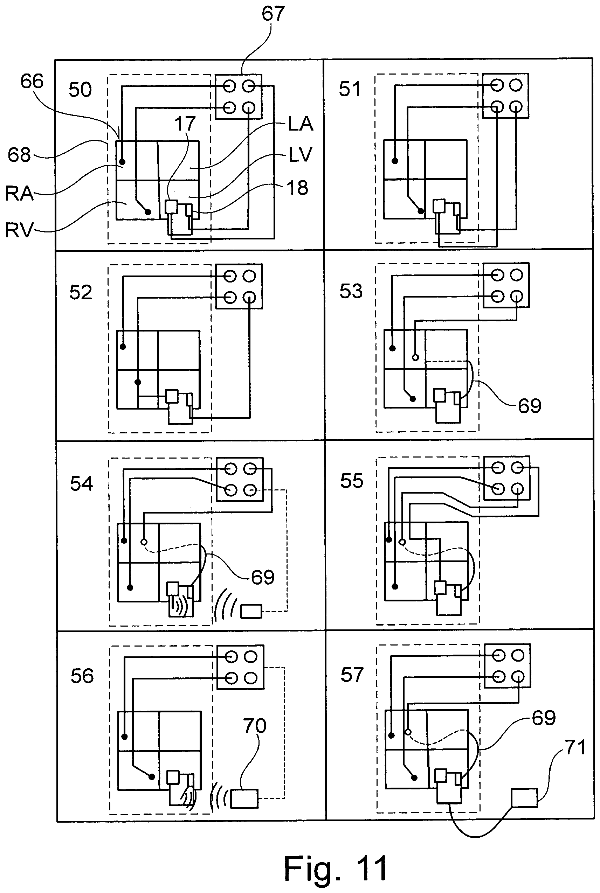

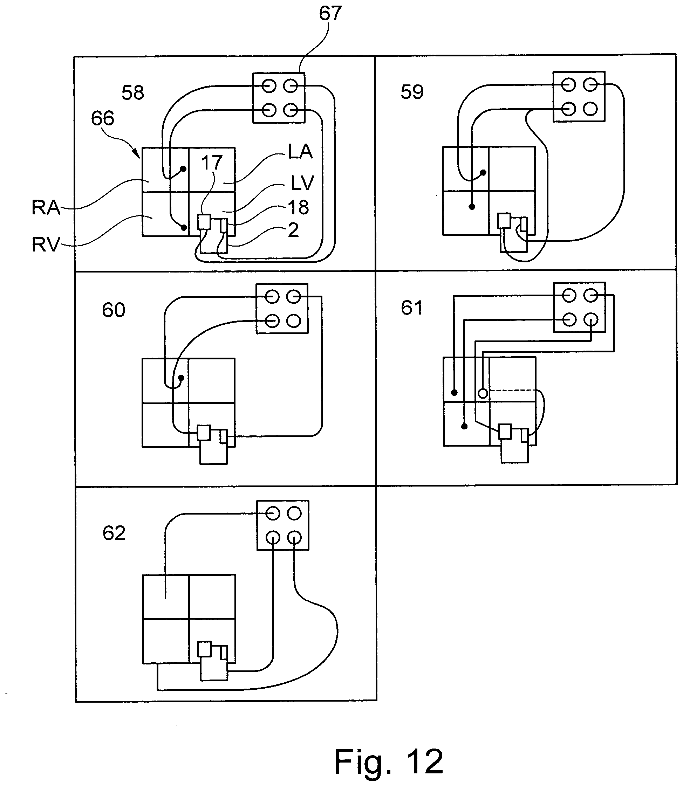

Die Figuren 11 und 12 stellen jeweils Gruppen von Anschlusskonzepten dar, wobei jedes Anschlusskonzept in einem gesonderten, nummerierten Rechteck gezeigt ist. Die Anschlusskonzepte 50 bis 57 sind aus Figur 11 ersichtlich, während die Anschlusskonzepte 58 bis 62 aus Figur 12 ersichtlich sind. In den Darstellungen der Anschlusskonzepte ist jeweils ein aus vier Quadranten bestehendes Rechteck gezeigt, das beispielhaft in Figur 11 im Konzept 50 mit dem Bezugszeichen 66 bezeichnet ist. Innerhalb jedes der Rechtecke 66 sollen die vier Quadranten gleich mit den Bezugszeichen RA (rechtes Atrium), RV (rechter Ventrikel), LA (linkes Atrium) und LV (linker Ventrikel) bezeichnet sein. Sie stellen die vier Herzkammern dar.

Im linken Ventrikel ist dabei jeweils ein Verschlusselement 2 mit zwei Elektroden 17, 18 dargestellt. Die Bezugszeichen sind nur in dem Konzept im Rechteck 50 eingezeichnet, sollen jedoch für alle dargestellten Anschlusskonzepte gelten, da in allen Konzepten auch das Verschlusselement 2 in gleicher Form wiedergegeben ist.

Mit dem Bezugszeichen 67 ist jeweils ein Gerät bezeichnet, das die Funktionen eines Herzschrittmachers, die Funktionen einer kardialen Resynchronisationstherapie sowie die Funktionen eines Kardioverters/Defibrillators haben kann. Jedoch kann das Gerät 67 auch nur einzelne oder jeweils zwei der genannten Funktionen in sich vereinigen, wobei zusätzliche Funktionen dann in einem getrennten Gerät wahrgenommen werden können.

Im Folgenden werden zu den einzelnen Konzepten stichpunktartig Besonderheiten und Funktionen erläutert. Das gestrichelt dargestellte Rechteck 68 bei den Anschlusskonzepten 50 bis 57 repräsentiert schematisch das Herz des Patienten und die innerhalb des Herzens angeordneten Sonden und Leitungen einschließlich der Leitungen, die durch Blutgefäße des Herzens geführt sind.

Figur 11:
Konzept 50: Die Sonden im rechten Ventrikel RV und Atrium RA sowie die Elektrode 18 sind mit dem Gerät 67 verbunden, um die Funktion einer kardialen Resynchronisationstherapie zu erfüllen. Die Elektrode 17 dient der Impulsgabe vom Gerät 67 zum Herzen in Erfüllung der Funktion eines implantierten Kardioverters/Defibrillators.
Konzept 51: Dieses Konzept sieht vor, dass die Sonde 17 mit demselben Anschluss im Gerät 67 verbunden ist wie die Sonde aus dem rechten Ventrikel, wobei der genannte Anschluss einerseits zur kardialen Resynchronisationstherapie und andererseits zur Abgabe von Impulsen in der Funktion eines Kardioverters/Defibrillators dient. Es kann eine Umschalteinrichtung zwischen Leitungen im oder am Gerät 67 vorgesehen sein.
Konzept 52: Im Unterschied zum Konzept 51 ist die Elektrode 17 unmittelbar durch die Herzwand mit der Sonde im rechten Ventrikel verbunden. Wie auch im Fall des Konzepts 51 können die Sonde im rechten Ventrikel und die Elektrode 17 sowohl die Funktion der kardialen Resynchronisationstherapie als auch die eines Kardioverters/Defibrillators realisieren.
Konzept 53: Wie Konzept 50, wobei jedoch die Zuleitung der Elektrode 18 durch den Sinus coronarius und Blutgefäße des Herzens hindurch zum Gerät 67 geführt ist.
Konzept 54: Die Elektrode 17 ist über eine nicht leitungsgebundene Übertragung mit dem Gerät 67 verbunden. Sie dient der kardialen Resynchronisationstherapie, ebenso wie die Elektrode 18, die über eine Leitung durch den Sinus coronarius, in den Figuren mit 69 bezeichnet, mit dem Gerät 67 verbunden ist. Die im rechten Ventrikel angeordnete Sonde dient der Funktion des Kardioverters/Defibrillators, während die Sonde im rechten Ventrikel der Resynchronisationstherapie dient.
Konzept 55: Gemäß diesem Konzept ist wiederum die Zuleitung der Elektrode 18 mittels einer Leitung durch den Sinus coronarius mit dem Gerät 67 verbunden und dient der Resynchronisationstherapie, ebenso wie die Sonde im rechten Atrium. Die Elektrode 17 ist mittels einer Leitung, die die Herzwand durchdringt, mit dem Gerät 67 verbunden und dient, ebenso wie die Sonde im rechten Ventrikel, der Kardioverter-/Defibrillatorfunktion.
Konzept 56: Dieses Konzept sieht vor, dass die Sonden im rechten Ventrikel und rechten Atrium mittels Leitungen mit dem Gerät 67 verbunden sind, während eine oder mehrere der Elektroden 17, 18 mittels eines in das Verschlusselement 2 integrierten Senders und eines Empfängers 70 mit dem Gerät 67 verbunden sind.
Konzept 57: Entsprechend diesem Konzept sind die Sonden im rechten Atrium und rechten Ventrikel mit dem Gerät 67 mittels Leitungen verbunden, und die Elektrode 18 am Verschlusselement ist mittels einer durch den Sinus coronarius führenden Leitung ebenfalls mit dem Gerät 67 verbunden. Zusätzlich ist eine Leitung dargestellt, die den Drucksensor am Verschlusselement mit einem Steuergerät 71 verbindet.

Figur 12:
Konzept 58: Gemäß diesem Konzept sind die Sonden im rechten Ventrikel und rechten Atrium mit dem Gerät 67 leitungsgebunden verbunden, ebenso wie die Elektroden 17 und 18, wobei die Elektrode 17 insbesondere der Funktion des Kardioverters oder Defibrillators dient.
Konzept 59: Im Unterschied zum Konzept 58 ist beim Konzept 59 vorgesehen, dass die Elektrode 17 mittels einer Leitung mit der Zuleitung der Sonde im rechten Ventrikel verbunden und mit dieser gemeinsam mit dem Gerät 67 verbunden ist. Die beiden Elektroden dienen insbesondere der Funktion des Kardioverters/Defibrillators. Der in der Darstellung durchgestrichene Anschluss an dem Gerät 67 wird nicht benötigt.
Konzept 60: Gemäß diesem Konzept ist die im rechten Atrium angeordnete Elektrode mit dem Gerät 67 über eine Leitung verbunden, ebenso wie die Elektrode 18 der Verschlusselements 2. Die Elektrode 17 des Verschlusselements ist mit dem Gerät 67 mittels einer Leitung verbunden, die transseptal durch den rechten Ventrikel hindurch verläuft.
Konzept 61: Gemäß dem Konzept 61 sind die Sonden im rechten Atrium und rechten Ventrikel mit dem Gerät 67 verbunden, die Elektrode 17 des Verschlusselements ist mittels einer transseptalen, durch den rechten Ventrikel führenden Leitung mit dem Gerät 67 verbunden, und die Elektrode 18 des Verschlusselements ist mittels einer durch den Sinus coronarius geführten Leitung ebenfalls mit dem Gerät 67 verbunden.
Konzept 62: Gemäß dem Konzept 62 ist die Sonde im oder am rechten Atrium mittels einer Leitung mit dem Gerät 67 verbunden. Eine Elektrode für den Defibrillator und / oder Sonde im Verschlusselement und eine Elektrode für eine rechtsventrikuläre Sonde werden extrakardial zum Gerät 67 geführt.

## Patentansprüche

1. Verschlusseinrichtung (1) für eine Öffnung (5) in einem Herz eines Patienten mit einem am Körpergewebe des Herzens im Bereich der Öffnung befestigbaren Ring (3, 3a, 3b) mit einer Ringöffnung und mit einem Verschlusselement (2), das zum Verschließen der Ringöffnung in diese einbringbar ist,
**dadurch gekennzeichnet, dass** das Verschlusselement (2) wenigstens einen integrierten Sensor (6, 10, 11, 16, 17, 18) und/oder wenigstens einen integrierten Aktor (17, 18) aufweist.

2. Verschlusseinrichtung nach Anspruch 1, **dadurch gekennzeichnet, dass** wenigstens ein integrierter Sensor (6, 16) zur Erfassung eines zeitabhängigen Drucks eingerichtet ist.

3. Verschlusseinrichtung nach Anspruch 2, **dadurch gekennzeichnet, dass** das Verschlusselement (2) wenigstens einen Drucksensor (6) aufweist, der einen Druck auf einer ersten Außenseite des Verschlusselements erfasst, sowie insbesondere einen zweiten Drucksensor (16), der auf einer zweiten Außenseite einen Druck erfasst, wobei das Verschlusselement derart eingerichtet ist, dass in der Verschlussposition des Verschlusselements die erste Außenseite dem Herzinneren und die zweite Außenseite dem Herzäußeren zugewandt ist.

4. Verschlusseinrichtung nach Anspruch 1, 2 oder 3, **dadurch gekennzeichnet, dass** das Verschlusselement (2) einen Sensor (10, 11, 17, 18) oder wenigstens zwei Sensoren zur Erfassung eines Spannungssignals aufweist.

5. Verschlusseinrichtung nach Anspruch 1, 2, 3 oder 4, **dadurch gekennzeichnet, dass** das Verschlusselement (2) wenigstens eine, insbesondere wenigstens zwei Elektroden (10, 11, 17, 18) aufweist, die in wenigstens einem oder wenigstens zwei Teilbereichen einen Oberflächenbereich des Verschlusselements (2), insbesondere einen zylindrischen oder teilzylindrischen Oberflächenbereich des Verschlusselements, bilden.

6. Verschlusseinrichtung nach Anspruch 1, 2, 3 oder 4, **dadurch gekennzeichnet, dass** das Verschlusselement (2) wenigstens eine, insbesondere wenigstens zwei Elektroden (10, 11, 17, 18) aufweist, die jeweils einen ersten, innerhalb des Verschlusselementes angeordneten Teil und einen zweiten, aus dem Verschlusselement herausragenden Teil aufweisen, wobei der zweite Teil jeweils eine Spitze (10a, 11a) aufweist.

7. Verschlusseinrichtung nach Anspruch 1, 2, 3, 4, 5 oder 6, **dadurch gekennzeichnet, dass** ein in das Verschlusselement integrierter Aktor eine Elektrode (10, 11, 17, 18) aufweist, die mit einem zeitabhängigen Spannungssignal beaufschlagbar und insbesondere mit einem Steuergerät (26, 27, 33, 36, 43, 45) verbindbar ist.

8. Verschlusseinrichtung nach Anspruch 1, 2, 3, 4, 5, 6 oder 7, **dadurch gekennzeichnet, dass** wenigstens ein Sensor und/oder mindestens ein Aktor (6, 10, 11, 16, 17, 18) mittels eines Kabels oder einer nicht leitungsgebundenen Übertragungseinrichtung direkt oder mittels einer in die Verschlusseinrichtung, insbesondere in das Verschlusselement, integrierten Vorverarbeitungseinrichtung (32, 38, 439 mit einem Steuer- oder Überwachungsgerät (26, 27, 33, 36, 43, 45) verbindbar ist.

9. Verschlusseinrichtung nach Anspruch 1,2, 3, 4, 5, 6, 7 oder 8, **dadurch gekennzeichnet, dass** wenigstens ein Sensor und/oder mindestens ein Aktor (6, 10, 11, 16, 17, 18) mit einem Energiespeicher (28) verbindbar ist, der entweder im Patientenkörper implantiert oder außerhalb des Patientenkörpers vorgesehen ist.

10. Verschlusseinrichtung nach Anspruch 9, **dadurch gekennzeichnet, dass** der Energiespeicher (28) in das Verschlusselement (2) oder in ein implantierbares/implantiertes Steuer- oder Überwachungsgerät (26, 27, 33, 36, 43, 45) integriert ist oder als Modul in den Patientenkörper implantiert ist.

11. Verschlusseinrichtung nach Anspruch 10, **dadurch gekennzeichnet, dass** der Energiespeicher (28) derart eingerichtet und angeordnet ist, dass er mittels einer außerhalb des Patientenkörpers angeordneten Induktionseinrichtung aufladbar ist.

12. Implantierbare Einrichtung mit einer Verschlusseinrichtung nach einem der vorangehenden Ansprüche sowie insbesondere mit einem Energiespeicher (28) und/oder einem Überwachungsgerät und/oder mit einem Steuergerät (26, 27, 33, 36, 43, 45).

13. Implantierbare Einrichtung nach Anspruch 12, **dadurch gekennzeichnet, dass** sie einen Herzschrittmacher (36) und/oder Defibrillator (43, 45) aufweist, der mit wenigstens einer in das Verschlusselement integrierten Elektrode verbunden ist.

14. Verfahren zum Betrieb einer implantierbaren Vorrichtung nach Anspruch 12 oder 13, **dadurch gekennzeichnet, dass** im implantierten Zustand mittels wenigstens eines in das Verschlusselement (2) integrierten Drucksensors (6, 16) ein Druckverlauf in einem, insbesondere dem linken Herzventrikel gemessen und hieraus laufend ein enddiastolischer Fülldruck bestimmt wird und/oder dass im implantierten Zustand mittels wenigstens einer in das Verschlusselement (2) integrierten Elektrode (17, 18) ein Signal oder Energieimpuls an den Herzmuskel abgegeben wird.
